# EUROPEAN PATENT SPECIFICATION

(11) **EP 1 602 381 B1**
(45) Date of publication and mention of the grant of the patent: **09.07.2008**
(21) Application number: 05010424.9
(22) Date of filing: 13.05.2005
(51) Int. Cl.: A61K 49/22

(54) **Contrast agent for combined modality imaging and methods and systems thereof**
KONTRASTMITTEL FÜR KOMBINIERTE ABBILDUNG UND DEREN SYSTEME UND VERFAHREN
AGENT DE CONTRASTE POUR IMAGERIE COMBINÉE ET LEURS METHODES ET SYSTEMES

(30) Priority: 14.05.2004 US 846062
(43) Date of publication of application: 07.12.2005
(73) Proprietor: GENERAL ELECTRIC COMPANY, Niskayuna New York 12309 (US)
(72) Inventor: Lomnes, Stephen Johnson, Albany New York 12210 (US); Uzgiris, Egidijus Edward, Niskayuna New York 12309 (US); Jansen, Flooribertus P. M., Ballston Lake New York 12308 (US); Fomitchov, Pavel Alexevevich, New York 10010 (US); Padilla de Jesus, Omayra Liz, Guliderland New York 12084 (US)
(74) Representative: Canning, Lewis R.

(56) References cited:
- WO-A-02/07779
- WO-A-02/22882
- WO-A-20/04057023
- US-A- 6 066 461
- US-A1- 2005 107 694
- ANTONY T ET AL: "MOLECULAR BEACONS: NUCLEIC ACID HYBRIDIZATION AND EMERGING APPLICATIONS" JOURNAL OF BIOMOLECULAR STRUCTURE & DYNAMICS, ADENINE PRESS, NEW YORK, NY, US, vol. 19, no. 3, December 2001 (2001-12), pages 497-504, XP009016988 ISSN: 0739-1102
- WALLRABE H ET AL: "Imaging protein molecules using FRET and FLIM microscopy" CURRENT OPINION IN BIOTECHNOLOGY, LONDON, GB, vol. 16, no. 1, February 2005 (2005-02), pages 19-27, XP004756192 ISSN: 0958-1669

## Description

### BACKGROUND

The invention relates generally to the field of diagnostic imaging and more specifically, to an imaging method and a system that uses contrast agents conjugated with dyes and quenchers for combined modality imaging, e.g., optical and ultrasound imaging.

In modem healthcare facilities, medical diagnostic and imaging systems are often used for identifying, diagnosing, and treating physical conditions. Diagnostic imaging refers to any visual display of structural or functional patterns of organs or tissues for a diagnostic evaluation. It includes measuring the physiologic and metabolic responses to either physical or chemical stimuli or a combination of the two. Currently, a number of modalities exist for medical diagnostic and imaging systems. These include ultrasound systems, optical imaging systems, computed tomography (CT) systems, x-ray systems (including both conventional and digital or digitized imaging systems), positron emission tomography (PET) systems, single photon emission computed tomography (SPECT) systems, and magnetic resonance imaging (MRI) systems- In many instances, final diagnosis and treatment proceed only after an attending physician or radiologist has complemented conventional examinations with detailed images of relevant areas and tissues via one or more imaging modalities.

Some of these imaging systems focus on imaging the molecular processes concomitant with a disease state rather than the anatomy of the subject. This type of imaging is generally referred to as molecular imaging. The subtle changes in physiological activities, which cause change in molecular concentrations of specific substance at the pico molar level, can provide early warning signs of diseases. Detecting such changes requires a highly sensitive imaging technique.

At present, a well-established way of molecular imaging uses nuclear medicine, where a radiopharmaceutical (that targets the specific target area) is injected into the patient. The decay of the radiopharmaceutical is used to construct an image of the bio-distribution of the agent. While this method is quite sensitive, it suffers from limited spatial resolution and anatomical registration, and has the further drawback of exposing the patient and the doctor to radiation.

ANTONY T ET AL: "MOLECULAR BEACONS: NUCLEIC ACID HYBRIDIZATION AND EMERGING APPLICATIONS" JOURNAL OF BIOMOLECULAR STRUCTURE & DYNAMICS, ADENINE PRESS, NEW YORK, NY, US, vol. 19, no. 3, December 2001 (2001-12), pages 497-504 discloses molecular beacons and its utility in fluorescence imaging techniques.

Optical imaging is an alternative form of molecular imaging that operates by passing light of certain wavelengths into a patient. For example, optical imaging generally operates in a near infrared part of the wavelength spectrum. The advantages of sub-surface optical imaging include the high-resolution visual images and the easy interpretability of the images. Still the scope of the technique has been largely limited to imaging the surface of the body. The limitations of this modality for the sub-surface imaging are due to light absorption and light scattering. For sub-surface imaging, optical imaging has relatively poor spatial resolution and anatomical registration.

In contrast to functional imaging, ultrasound imaging is a well-established modality for quickly obtaining images of a patient's anatomy. In operation, an ultrasound imaging system transmits an ultrasound wave into a subject and subsequently receives a reflected wave that is generated at the interface between tissues of different acoustic impedance. The position of the tissue can be calculated based on the time of arrival and approximate velocity of the reflected wave. Thus, ultrasound imaging systems can identify the shape and position of certain anatomies. While there are inherent advantages in the technique such as reduced patient wait time, faster examination procedure, etc., the primary disadvantage of the technique is the high noise of images. For this reason, the interpretation of the images for a proper diagnosis requires considerable skill.

In view of the advantages and disadvantages of these different imaging modalities, a technique is needed for combining the high molecular sensitivity of functional imaging modalities (e.g., optical imaging) with the spatial resolution of anatomical imaging modalities (e.g., ultrasound).

### BRIEF DESCRIPTION

Briefly, in accordance with one aspect of the present invention, a contrast agent for a combined modality imaging system includes a deformable particle that has a geometry that changes in response to an emission from the combined modality imaging system. The deformable particle also includes a fluorescent component that is adapted to emit electromagnetic radiation and a quenching component separated from the fluorescent component and adapted to absorb a portion of the electromagnetic radiation from the fluorescent component.

In accordance with another aspect of the present invention, a combined modality imaging system includes a first imaging device of a first modality and a second imaging device of a second modality that is different from the first modality. The first and the second imaging devices are both adapted to interact with a contrast agent adapted to be received in a subject. The contrast agent includes a deformable particle that has a geometry that varies in response to an emission from the first imaging device. The deformable particle also includes a fluorescent component adapted to emit electromagnetic radiation that is detectable by the second imaging device and a quenching component separated from the fluorescent component at a distance based on the geometry and that is adapted to absorb a portion of the electromagnetic radiation from the fluorescent component.

In accordance with another aspect of the present invention, the use of the contrast agent in the combined modality imaging system includes disposing a subject under diagnosis with a solution of a contrast agent that includes a deformable particle. The deformable particle includes a fluorescent component adapted to emit electromagnetic radiation detectable by an electromagnetic radiation based imaging device and a quenching component that is separated from the fluorescent component at a distance based on a geometry of the deformable particle, wherein the quenching component is adapted to absorb a portion of the electromagnetic radiation emitted by the fluorescent component. The quenching component can also effect an energy transfer without emission of electromagnetic radiation from the fluorescent component by a fluorescent resonance energy transfer mechanism. The method of use of the combined modality imaging system also includes applying ultrasound waves from an ultrasound imaging system on to a region of interest of an ultrasound probe in a region of interest on the subject, applying electromagnetic radiation using an electromagnetic excitation source on the region of interest, detecting the reflected ultrasound signals using the ultrasound probe, detecting the electromagnetic radiation from the contrast agent using an electromagnetic radiation detector, processing the detected ultrasound signals and the electromagnetic radiation to obtain two separate but co-registered images and finally displaying the images from the combined modality imaging system.

### DRAWINGS

These and other features, aspects, and advantages of the present invention will become better understood when the following detailed description is read with reference to the accompanying drawings in which like characters represent like parts throughout the drawings, wherein:

FIG. 1 is a diagrammatical representation of a combined modality imaging system according to aspects of present technique;

FIG. 2 is a diagrammatical representation of an ultrasound imaging system for use in the multiple modality imaging system of Fig. 1;

FIG. 3 is a diagrammatical representation of an optical imaging system for use in the multiple modality imaging system of Fig.1;

FIG. 4 is a diagrammatical representation of an alternate implementation of a combined modality imaging system, wherein a single unit comprises an ultrasound probe, an electromagnetic excitation source at one side of the ultrasound probe, and an electromagnetic radiation detector at an opposite side of the ultrasound probe;

FIG. 5 is a diagrammatical representation of another alternate implementation of a combined modality imaging system, wherein a single unit comprises the ultrasound probe and electromagnetic radiation detectors located at opposite sides of the ultrasound probe;

FIG. 6 is a diagrammatic representation of an embodiment of a contrast agent for use with a multiple modality imaging system, wherein a multitude of fluorescent component - quenching component pairs are attached to the outer surface of a deformable particle;

FIG. 7 is a diagrammatic representation of an alternate embodiment of the contrast agent for use with a multiple modality imaging system, wherein a multitude of fluorescent component - quenching component pairs are attached to the inner surface of a deformable particle;

FIG. 8 is a diagrammatic representation of another alternate embodiment of the contrast agent for use with a multiple modality imaging system, wherein a multitude of fluorescent and quenching components are disposed within a shell of a deformable particle;

FIG- 9 is a diagrammatic representation of a further embodiment of the contrast agent for use with a multiple modality imaging system, wherein a multitude of fluorescent and quenching components are disposed in individual shells contained one within the other about a central compressible core;

FIG. 10 is a diagrammatic representation illustrating the interaction between ultrasound waves and a single contrast agent particle disposed within a subject in accordance with embodiments of the present technique;

FIG- 11 is a diagrammatic representation illustrating the interaction between the electromagnetic radiation, and a single contrast agent particle disposed within a subj ect in accordance with embodiments of the present technique;

FIG. 12 is a diagrammatic representation illustrating the combined interaction between ultrasound waves, electromagnetic radiation, and a single contrast agent particle disposed within a subject in accordance with embodiments of the present technique;

FIG. 13 is a flowchart illustrating an exemplary method of use of a combined modality imaging system in accordance with embodiments of the present technique; and

FIG. 14 is a flowchart illustrating an exemplary method of operation for a contrast agent according to aspects of the present technique.

### DETAILED DESCRIPTION

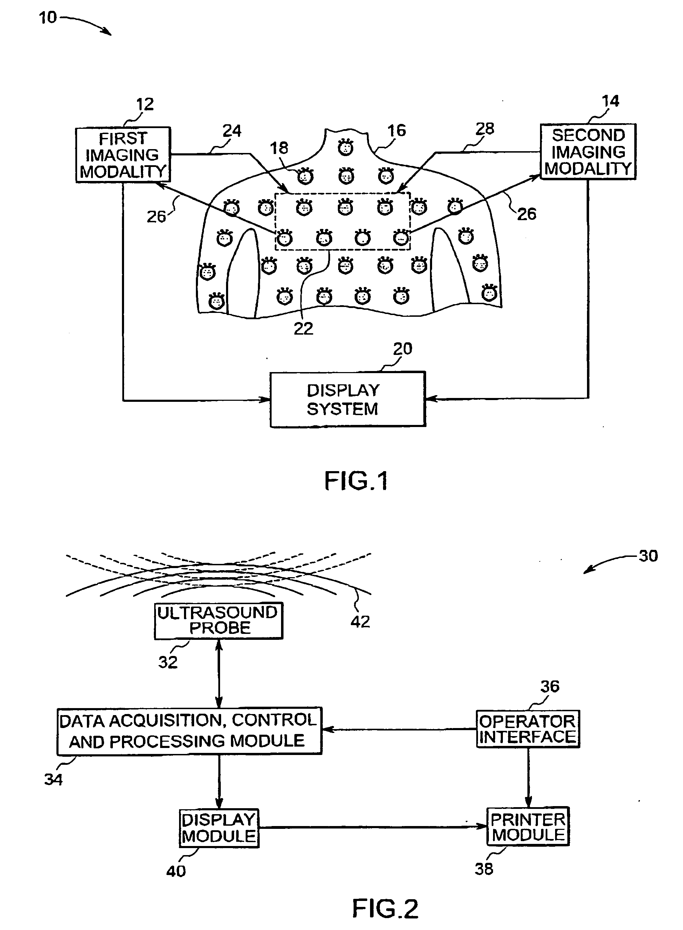

Turning now to the drawings, and referring first to FIG. 1, a combined modality imaging system 10 is illustrated schematically as including a first imaging modality 12, a second imaging modality 14, a subject 16 injected with a solution of a contrast agent 18, and a display system 20 capable of displaying the image from the first and second imaging modalities. The contrast agent 18 generally accumulates in regions of interest, such as tumors, to enhance imaging of those regions of interest. As discussed in detail below, certain embodiments of the contrast agent 18 comprise a deformable particle having a fluorescent component and a quenching component offset from the fluorescent component, such that variation in the geometry of the deformable particle changes the distance between the fluorescent and quenching components. As a result, the electromagnetic radiation emitted from the contrast agent 18 varies with distance between the fluorescent and quenching components. A greater distance results in relatively more emitted electromagnetic radiation and a smaller distance results in relatively less radiation. In operation, this correlation between radiation emission and distance, also known as Forster resonance energy transfer, facilitates localization of the radiation emitted from the contrast agent 18.

According to aspects of the present technique, the first imaging modality 12 focuses pressure waves 24 at a desired frequency onto a region of interest 22 on the subject 16 and retrieves reflected pressure waves 26 from the region of interest 22 to obtain an image. For example, one embodiment of the first imaging modality 12 includes an ultrasonic probe 32 that transmits and receives ultrasound waves in a region of interest 22. In the region of interest 22, the pressure waves 24 function to alter the geometry of the contrast agent 18, thereby modulating the fluorescence emitted by the contrast agent 18 at the frequency of the pressure waves 24. Embodiments of the second imaging modality 14 detect this fluorescent modulation to generate an optical molecular image which is substantially localized based on the focus area 22. In operation, the second imaging modality 14 transmits electromagnetic radiation 28 onto the region of interest 22 and then utilizes the interaction between the first imaging modality 12, the contrast agent 18, and the electromagnetic radiation 28 to generate an image. The display system 20 can display the images from the two different modalities either separately or as a composite image where the images are superimposed one on top of the other. The present technique combines the advantages of a high molecular sensitivity of functional imaging modalities (e.g., optical imaging) with the advantages of a high spatial resolution of anatomical imaging modalities (e.g., ultrasound imaging) to improve image quality and diagnosis.

FIG. 2 illustrates an exemplary first imaging modality 12 as an ultrasound system 30 that includes an ultrasound probe 32, a data acquisition and processing module 34, an operator interface 36, a printer module 38, and a display module 40.

In operation, the ultrasound probe 32 sends and receives ultrasound waves 42 from a region of interest on the subject 16. The ultrasound probe 32, according to aspects of present technique, includes at least one of an ultrasound transducer, a piezoelectric crystal, an opto-acoustic transducer and a micro electro mechanical system device, e.g., a capacitive micro-machined ultrasound transducer (cMUT). As appreciated by those of ordinary skill in the art of ultrasound, the ultrasound waves 42 obey the laws of geometric optics with regard to transmission, reflection, and refraction. The relatively high frequency of ultrasound also facilitates relatively focused targeting of the ultrasound waves 42. During the operation of the ultrasound system 30, the ultrasound waves 42 reflected from the subject carry information about the thickness, size, and location of various tissues, organs, tumors, and anatomical structures in relation to the transmitted ultrasound wave. In certain embodiments, the ultrasound probe 32 can be band-held or mechanically positioned using a robotic assembly.

The data acquisition, control and processing module 34 sends and receives information from the ultrasound probe 32. It controls the strength, the width, the duration, and the frequency of the ultrasound waves 42 transmitted by the ultrasound probe 32 and decodes the information contained in the ultrasound waves 42 reflected from the focus area 22 to discernable electrical and electronic signals. Once the information is obtained, the image of the object located within the region of interest 22 of the ultrasound probe 32 is reconstructed.

The operator interface 36 may include a keyboard, a mouse, and other user interaction devices. The operator interface 36 can be used to customize the settings for the ultrasound examination, and for effecting system level configuration changes. The operator interface 36 is connected to the data acquisition, control and processing module 34 and to the printer module 38. The printer module 38 is used to produce a hard copy of the obtained ultrasound image in either gray-scale or color. The display module 40 presents the reconstructed image of an object within the region of interest 22 on the subject 16 based on data from the data acquisition and processing module 34.

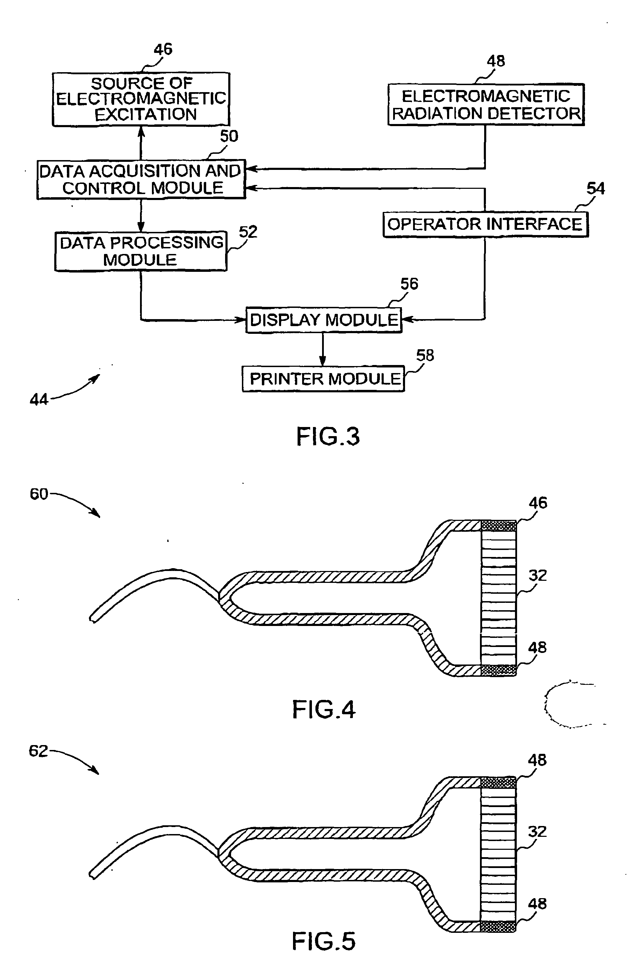

FIG. 3 illustrates an exemplary optical imaging system 44 in accordance with embodiments of the present technique. In certain embodiments, the optical imaging system 44 operates in conjunction with the ultrasound imaging system 30 of Fig. 2- The illustrated optical imaging system 44 includes an electromagnetic excitation source 46, an electromagnetic radiation detector 48, a data acquisition and control module 50, a data processing module 52, an operator interface 54, a display module 56, and a printer module 58. As discussed in further detail below, the optical imaging system 44 records the interaction between the ultrasound system 30, a solution of the contrast agent 18 injected within and located in the region of interest 22 of the subject 16, and the electromagnetic radiation from the electromagnetic excitation source 46.

The illustrated electromagnetic excitation source 46 has at least one of a solid state light emitting diode (LED), an organic light emitting diode (OLED), a laser, an incandescent lamp, a halogen lamp, an arc lamp and any other suitable light source. For example, the electromagnetic excitation source 46 may emit radiation between the ranges of about 300 nanometers and about 2 micrometers that is matched to the absorption wavelength of a fluorescent component. Certain embodiments of the electromagnetic excitation source 46 emit electromagnetic radiation whose intensity can be time invariant, a sinusoidal variation, a pulse variation, or time varying. The electromagnetic radiation can also comprise a single wavelength or many wavelengths covering a spectrum from about 300 nanometers to about 2 micrometers- Fiber-optic channels, such as an optic fiber and bundles of optic fibers can also be used to provide illumination from the electromagnetic excitation source 46 to the focus area 22.

The illustrated electromagnetic radiation detector 48 has at least one of a photomultiplier tube, a charged-coupled device, an image intensifier, a photodiode, an avalanche photodiode, and any suitable device that can convert a tune-varying flux of electromagnetic radiation to a time-varying electrical signal. An array of optical fibers can also be extended from the electromagnetic radiation detector 46 to the vicinity of the focus area 22 to collect electromagnetic radiation. For example, the optical fibers may be mounted either directly on the subject 16 or near the surface of the subj ect 16.

The illustrated data acquisition and control module 50 sends control signals to the electromagnetic excitation source 46 and receives the optical signals from the electromagnetic radiation detectors 48. The data acquisition and control module 50 also communicates with the data processing module 52 and the user interface module 54. The data processing module 52 re-constructs an image using the information obtained from the electromagnetic radiation detector 48. The user interface module 54 is used to make changes to the configuration of the optical imaging system 44 and to provide control commands to the display module 56 and the printer module 58.

In certain embodiments, the combined modality imaging system 10 includes the functionalities of both the ultrasound and the optical imaging systems as described in detail above. FIGS- 4 and 5 are exemplary embodiments of such combined modality imaging systems. The embodiment of FIG. 4 comprises a single unit having the ultrasound probe 32 of the ultrasound imaging system 30 located at the center of the single unit, and the electromagnetic excitation source 46 and the electromagnetic detector 48 of the optical imaging system 44 located at opposite sides of the single unit. The embodiment of FIG. 5 comprises a single unit having the ultrasound probe 32 of the ultrasound imaging system 30 at the center of the single unit, and a pair of the electromagnetic radiation detectors 48 of the optical imaging system 44 at opposite sides of the ultrasound probe 32.

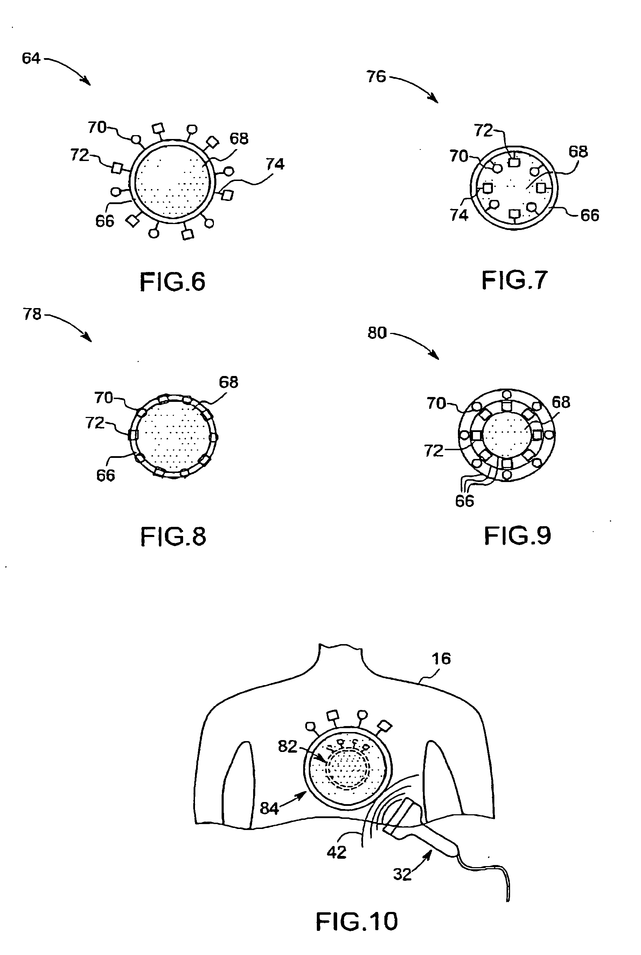

As described below with reference to FIGS- 6-9, the foregoing imaging systems 10, 30, and 44 interact with a variety of different embodiments of contrast agents. FIG. 6 is a diagrammatic illustration of an embodiment 64 of the contrast agent 18 that comprises a deformable particle, including a shell 66 and an internal substance 68 disposed within the shell 66. The deformable particle also includes one or a multitude of fluorescent component 70 - quenching component 72 pairs, each component attached to the outer surface of the deformable particle. FIG. 7 is a diagrammatic illustration of an alternate embodiment 76 of the contrast agent 18 comprising a deformable particle including a shell 66 and an internal substance 68 disposed within the shell 66. The deformable particle also includes at least one of a fluorescent component 70 - quenching component 72 pair disposed within the shell 66 of the deformable particle, each component attached to the inner surface of the deformable particle by means of a linker component 74. FIG. 8 is a diagrammatic illustration of another alternate embodiment 78 of the contrast agent 18 that comprises a deformable particle, wherein a multitude of the fluorescent component 70 - quenching component 72 pairs form the shell 66 of the deformable particle. FIG. 9 is a diagrammatic illustration of a further embodiment 80 of the contrast agent 18, wherein at least of the fluorescent component 70 and the quenching component 72 is disposed separately in multiple layers of the deformable particle and the inner shell comprises a compressible core. In each of the foregoing embodiments, the sound wave 42 (e.g., an ultrasound wave) changes the geometry of the deformable particle, thereby changing the distance between the fluorescent component - quenching component pairs. The figures discussed below further describe the composition and the interaction of the contrast agents 18 with the ultrasound imaging system 30 illustrated in FIG. 2 and the optical imaging system 44 illustrated in FIG. 3.

The shell 66 of the deformable particle includes at least one of a polymer, a protein, and an amphiphilic molecule. An amphiphilic substance is one that has both hydrophobic and hydrophilic regions, such as surfactants. The term applies to small molecules such as phospholipids. The amphiphilic molecule includes at least one surfactant of an ionic nature or a non-ionic nature, wherein the surfactant includes at least one functional group that provides a reactive handle for a continued chemical modification. The components of shell 66 comprise at least one functional group containing reactive handles for further chemical modification. The internal substance 68 disposed within the shell 66 is compressible, and in certain embodiments, may include at least one of air, sulfur hexafluoride, a perfluorocarbon, foam, a gas precursor and polymer. The fluorescent component 70 comprises a fluorescent dye. For example, the fluorescent dye may include indocyanine green (ICG), cyanine 5.5 (CY5.5), cyanine 7.5 (CY7.5), fluorescein, rhodamine, yellow fluorescent protein (YFP), green fluorescent protein (GFP), fluorescein isothiocyanate (FITC) and their derivatives. The quenching component 72 comprises at least one of known quenching entities and derivatives thereof. The aforementioned fluorescent component can be self-quenching at a suitable molecular concentration and separation level characteristic for that fluorescent component. The contrast agent 18 could also include a chemical substance conjugated to the deformable particle, where the chemical substance binds preferentially to a specific biochemical marker. This means that the contrast agent can actively target an abnormal tissue by means of preferential accumulation based on the differences between the abnormal tissue and a normal tissue. For example, the biochemical marker may include a monoclonal antibody, fragments of an antibody e.g., fowl adenovirus (FAV), constructs of an antibody, a polypeptide, other small organic molecules and combinations thereof.

The fluorescent component 70 has a property wherein it absorbs electromagnetic radiation at an incident wavelength and emits electromagnetic radiation at a longer wavelength. The quenching component 72 has a property wherein it absorbs electromagnetic radiation at the wavelength emitted by the fluorescent component 70. One function of the fluorescent component 70 is to maximize the light output from the focus area 22 of the ultrasound probe 32. One function of the quenching component 72 is to maximize the signal-to-noise ratio by minimizing the intensity of fluorescent light produced by particles that are not near the region of interest on the subject 16.

If the distance between the fluorescent component 70 and the quenching component 72 is less than a characteristic distance and the electromagnetic radiation from the electromagnetic excitation source 46 is incident on the region of interest on the subject 16, then the electromagnetic radiation emitted by the fluorescent component 70 (after absorbing the incident electromagnetic radiation from the electromagnetic excitation source 46 illustrated in FIG. 3) is quenched by the quenching component 72. Quenching occurs when the quenching component 72 absorbs most of the electromagnetic radiation emitted by the fluorescent component 70. Quenching can also occur by a fluorescent resonance energy transfer mechanism where the quenching component 72 absorbs the energy from the fluorescent component 70 without any emission of electromagnetic radiation from the fluorescent component 70. As a result, there is a weak output of light from the contrast agent 18 that is insufficient to be detected by the electromagnetic radiation detector 48. At this point, the contrast agent 18 is said to be in an OFF state. A typical dimension of the contrast agent in its OFF state is less than 15 micrometers in diameter. If the distance of separation between the fluorescent component 70 and the quenching component 72 at least exceeds the characteristic distance, called the Forster distance, and the electromagnetic radiation from the electromagnetic excitation source 46 is incident on the focus area 22 of the subject 16, then the electromagnetic radiation emitted by the fluorescent component 70 would not be absorbed by the quenching component 72 and there is light output from the contrast agent 18. At this state, the contrast agent 18 is said to be in an ON state.

The increase in the distance of separation between the fluorescent component 70 and the quenching component 72 is effected when the contrast agent 18 is subjected to ultrasound waves 42 from the proposed ultrasound imaging system 30 illustrated in FIG. 2. Under the influence of acoustic pressure, such as ultrasound waves 42 from an ultrasound imaging system 30, the contrast agent 18 undergoes a change in geometry. In certain embodiments, the ultrasound waves 42 increase the volume of the contrast agent 18. Due to the pulsed nature of the ultrasound waves 42, the contrast agent 18 undergoes repeated compression and expansion resulting in a volume change, which can be of the order of 300% in certain embodiments. The change in volume causes a change in the distance of separation between the fluorescent component 70 and the quenching component 72. Accordingly, there is modulation of the light output every time an ultrasound wave 42 interacts with the contrast agent 18- Therefore, this light output enables the data acquisition and control module 50 of the proposed optical imaging system 44 to collect optical data through the electromagnetic radiation detectors 48 and to process the optical data with the data processing module 52. The data processing module 52 of the optical imaging system 44 computes this optical data to obtain an optical image that is co-registered with the ultrasound image from the ultrasound system 30 illustrated in FIG. 2.

FIG. 10 is an exemplary illustration of interaction between ultrasound waves 42 from the ultrasound imaging system 30 and a single contrast agent 18 in accordance with embodiments of the present technique. Before the ultrasound wave 42 hits the contrast agent 18, the contrast agent 18 is in its ground or unexcited state 82, wherein the distance of separation between the fluorescent component 70 and the quenching component 72 is less than the characteristic distance. When the ultrasound waves 42 hits the contrast agent 18, due to the foregoing characteristics of the contrast agent 18, the contrast agent 18 undergoes a change in geometry that increases the distance of separation between the fluorescent component 70 and the quenching component 72. At this stage, the contrast agent 18 is in an excited stage 84, wherein the distance of separation between the fluorescent component 70 and the quenching component 72 at least exceeds the characteristic distance.

FIG-11 is an exemplary illustration of interaction between a single contrast agent 18 and an electromagnetic excitation source 46 from the optical imaging system 44 in accordance with embodiments of the present technique. The electromagnetic excitation source 46 emits electromagnetic radiation 86 between ranges of about 300 nanometers and about 2 micrometers and matched to the absorption wavelength of the contrast agent 18. The fluorescent component 70 absorbs the incident electromagnetic radiation 86, and emits electromagnetic radiation 88 at a longer wavelength. However, since the contrast agent 18 is in its unexcited state 82, the distance between the fluorescent component 70 and the quenching component 72 is less than the characteristic distance and there is maximum energy transfer between the two components. Because there is maximum energy transfer, the quenching component 72 absorbs the electromagnetic radiation 88 emitted by the fluorescent component 70 and there is a weak output in the form of an electromagnetic radiation from the contrast agent 18.

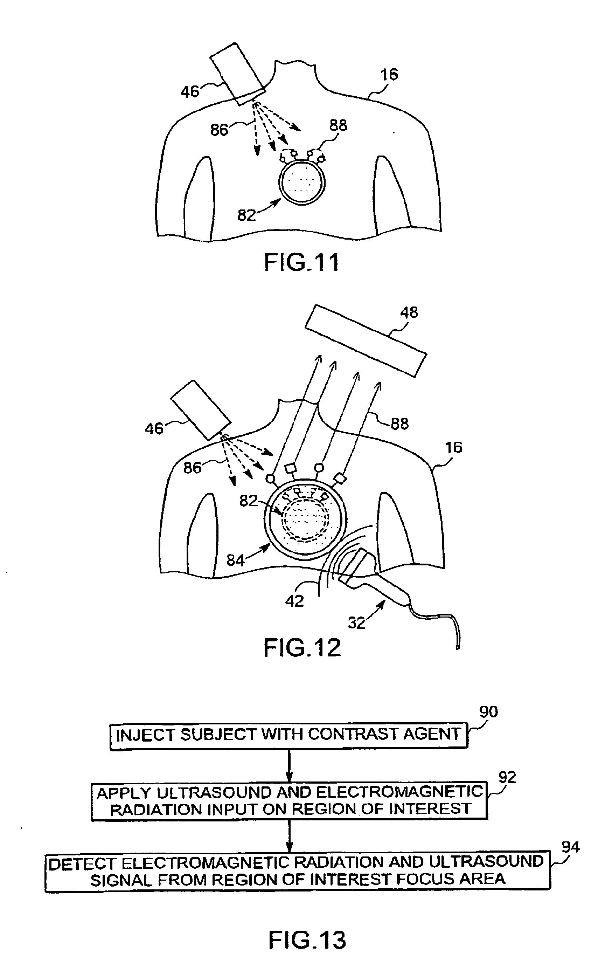

FIG. 12 illustrates the combined interaction of the ultrasound and optical imaging modalities described hereinabove with a contrast agent 18 in accordance with embodiments of the present technique. In operation, the electromagnetic radiation 86 from the electromagnetic excitation source 46 is incident on a contrast agent 18 in the focus area 22 of an ultrasound probe 32. First, the ultrasound waves 42 from an ultrasound probe 32 strike the contrast agent 18, thereby causing a change in the state of the contrast agent 18 from an OFF state 82 to an ON state 84, resulting in an expansion of the deformable particle of the contrast agent 18. As discussed above, the expansion causes an increase in the distance of separation between the fluorescent component 70 and the quenching component 72. Because the electromagnetic radiation 86 is incident on the fluorescent component 70 of the excited contrast agent 84, the electromagnetic radiation detector 48 of the optical imaging system 44 detects the output in the form of an electromagnetic radiation 88 emitted by the contrast agent 18. In an alternative embodiment of the present technique, the contrast agent 18 may behave differently when subjected to an ultrasound pulse as discussed below. Consider when the contrast agent is subjected to an ultrasound pulse. Specifically, in this alternative embodiment, the contrast agent 18 may change geometry in a manner in which the volume of the contrast agent increases with each ultrasound wave that passes through the contrast agent 18. When the ultrasound wave 42 is turned off, the volume of the contrast agent 18 does not shrink back to its original state abruptly. Instead, the volume of the contrast agent 18 undergoes a gradual reduction in its geometry until its ground state is reached.

FIG. 13 illustrates an exemplary method of use of the combined modality imaging system 10 illustrated in FIG. 1 in accordance with embodiments of the present technique. The method involves injecting the subject 16 with a contrast agent 18 at step 90. After a sufficient amount of time, the contrast agent 18 flows through the subject 16 to the region of interest 22, where the imaging is to be performed to aid in a diagnosis. At step 92, the inputs (ultrasound waves and electromagnetic radiation) from the combined modality imaging system 10 are applied onto the focus area 22 of the subject 16 as described herein above. The contrast agent 18 interacts with both the ultrasound imaging system 30 and the optical imaging system 44 in the manner described in the sections herein above. At step 94, the combined modality imaging system 10 detects the electromagnetic radiation emitted by a multitude of the fluorescent component 70 of the contrast agent 18 as well as the ultrasound waves 42 reflected from the focus area of the subject- In one embodiment, this enables a simultaneous mapping of the radiographic ultrasound image obtained from the ultrasound imaging system 12 with the concentration of the contrast agent, which is measured by an intensity of electromagnetic radiation emitted by the contrast agent 18 and detected by electromagnetic radiation detectors 48 in the optical imaging system 14. This intensity of electromagnetic radiation can be the modulated intensity as received or it can be a modified intensity based on an estimate of the attenuation caused by any intermediate tissue or organ. Finally, at step 96, the co-registered images from the first imaging modality 12 and the second imaging modality 14 are displayed. The display can be separate displays or a composite display wherein the images from the two different modalities are superimposed one over the other.

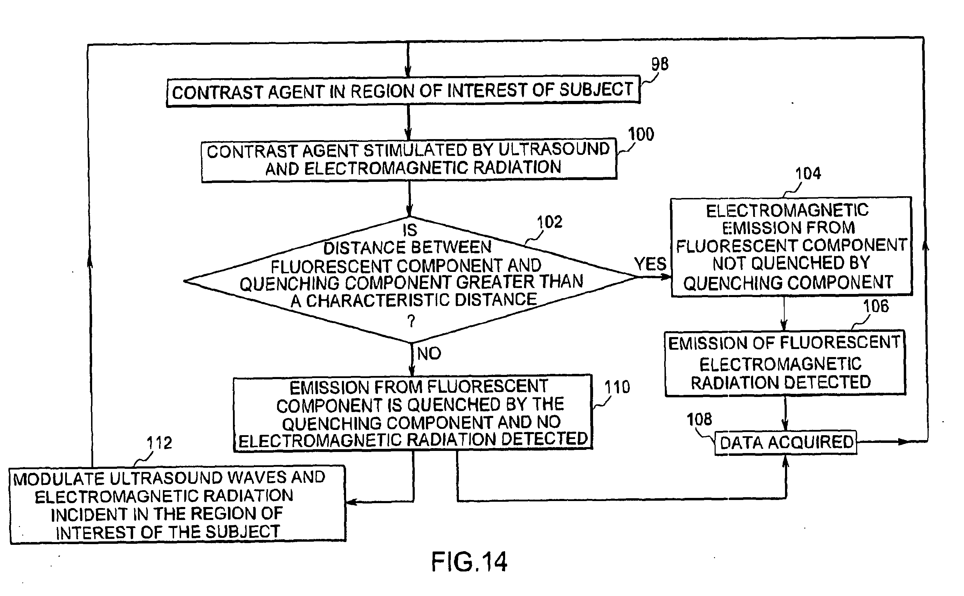

FIG. 14 illustrates a method of operation for a contrast agent (e.g., as illustrated in FIGS. 6-9) and combined modality imaging system in accordance with certain embodiments of the present technique. The contrast agent comprises a deformable particle having a geometry that varies in response to emission from a combined modality imaging system (e.g., as illustrated in FIG- 1). The deformable particle includes at least one of a fluorescent component and a quenching component.

At step 98, the contrast agent 18 initially accumulates in a region of interest 22 on a subject 16. At step 100, the contrast agent 18 excites or becomes stimulated in response to ultrasound and electromagnetic radiation. For example, an input in the form of an electromagnetic radiation 28 from the combined modality imaging system 10 may be applied on the region of interest 22 containing the contrast agent 18, such that there is emission of electromagnetic radiation from the fluorescent component 70. The quenching component absorbs a portion of the electromagnetic radiation emitted by the fluorescent component 70- As discussed in detail above, the amount of absorption depends on the distance of separation between the fluorescent component and the quenching component. The distance of separation is governed by the geometry of the deformable particle.

Furthermore, at step 100, when an input in the form of an ultrasound wave is directed towards the region of interest 22, the deformable particle undergoes a change in geometry that results in a change in the distance of separation between the fluorescent component and the quenching component. Step 102, represents the dependence on the distance of separation as a factor that determines whether the contrast agent 18 emits electromagnetic radiation or not. The flow proceeds to step 104 if the distance of separation at least equals a characteristic distance, called the Forster distance. The fluorescent component 70 emits electromagnetic radiation that is not absorbed by the quenching component 72. As shown in step 106, the contrast agent 18 emits electromagnetic radiation detectable by an electromagnetic radiation detector. If the distance of separation is less than the Forster distance, then the flow proceeds from step 100 to step 110. During this phase, the emitted electromagnetic radiation from the fluorescent component is quenched by the quenching component by any one of the quenching mechanisms described in detail above,

At step 112, the ultrasound wave 32 from the combined modality imaging system can be suitably modified to increase the distance of separation. Furthermore, at step 112, the wavelength of the electromagnetic radiation from the electromagnetic excitation source 46 can be modified to facilitate maximum absorption by the fluorescent component. Step 108 represents the continuous acquiring of data irrespective of whether there is emission of electromagnetic radiation from the contrast agent. The process is repeated until sufficient data has been acquired.

In accordance with certain embodiments of the present technique, a method of manufacture of a contrast agent (e.g., as illustrated in FIGS 6-9) may comprise the steps discussed in detail below. The contrast agent 18 includes a deformable particle that has a shell 66 and an internal substance 68 along with at least one of a fluorescent component 70 and a quenching component 72. The method involves using a template as a temporary core that facilitates the manufacture of contrast agents of uniform dimension. In certain embodiments, the shell 66 may be assembled on top of the template by the formation of covalent bonds, such as covalent bonds made by a cross-linking by partial denaturation of a protein, a cross-linking with a polyfunctional linker, cross-linking with a polymerizable group, and any combinations thereof. Alternatively, in other embodiments, the shell 66 may be stabilized by at least one non-covalent interaction, such as a hydrophobic interaction, a hydrophilic interaction, and an ionic interaction. The covalent bond has at least one of a biodegradable linkage and a non-biodegradable linkage. The template is then removed from the stabilized shell by at least one of a chemical conditioning process, and heating. The deformable particle is thus formed. Individual components containing functional handles that allow for further modification of the deformable particle are introduced. These functional handles facilitate the attachment of the fluorescent component 70 and the quenching component 72 to the shell 66. Alternately, in another embodiment, the fluorescent component 70 and the quenching component 72 can attach directly to the shell 66. One of the fluorescent component 70 and the quenching component 72 are introduced to the deformable particle for the formation of the contrast agent 18.

## Claims

1. A contrast agent (18) for an imaging system (10), comprising:
a deformable particle having a geometry that varies in response to an emission from the imaging system, the deformable particle comprising:
a shell (66) having an internal substance (68);
a fluorescent component (70) adapted to emit electromagnetic radiation (28); and
a quenching component (72) separated from the fluorescent component (70) at a distance based on the geometry, wherein the quenching component (72) is adapted to absorb a portion of the electromagnetic radiation (28) from the fluorescent component (70).

2. The contrast agent (18) of claim 1, wherein the shell (66) comprises a multitude of the fluorescent component (70) and the quenching component (72).

3. The contrast agent (18) of claim 1, wherein the internal substance (68) comprises the fluorescent component (70) and the quenching component (72).

4. The contrast agent (18) of claim 1, wherein the shell (66) comprises one of the fluorescent component (70) or the quenching component (72) and the internal substance (68) comprises a remaining one of the fluorescent component (70) or the quenching component (72).

5. A combined modality imaging system (10), comprising:
a first imaging device of a first modality (12); and
a second imaging device of a second modality (14) different from the first modality (12), wherein the first and the second imaging devices are both adapted to interact with a contrast agent (18) adapted to be received in a subject (16), the contrast agent (18) comprising:
a shell (66) having an internal substance (68);
a deformable particle having a geometry that varies in response to an emission from the first imaging system, the deformable particle comprising:
a fluorescent component (70) adapted to emit electromagnetic radiation (28) detectable by the second imaging system; and
a quenching component (72) separated from the fluorescent component (70) at a distance based on the geometry, wherein the quenching component (72) is adapted to absorb a portion of the electromagnetic radiation (28).

6. The combined modality imaging system (10) of claim 5, wherein the first imaging device comprises an ultrasound imaging device (30).

7. The combined modality imaging system (10) of claim 5, wherein the second imaging device comprises an optical imaging device (44).

8. Use of the the contrast agent (18) of claim 1, in the combined modality imaging system of claim 6, said use comprising:
allowing sufficient time for said contrast agent (18) to flow through a subject (16) to a region of interest (22) within said subject (16);
emitting electromagnetic radiation (28) from the fluorescent component (70) in response to emissions from an electromagnetic radiation based imaging device;
increasing the geometry of the deformable particle in response to a pressure wave (24) by an ultrasound imaging device (30); and
decreasingly absorbing, with the quenching component (72), a portion of the electromagnetic radiation (28) emitted by the fluorescent component (70) in response to increasing the geometry of the deformable particle.

9. A method of manufacturing a contrast agent (18) for an imaging system, the method comprising:
building a shell (66) around a temporary core;
stabilizing the shell (66);
removing the temporary core to form a deformable particle of a contrast agent (18) said deformable particle having a geometry that varies in response to an emission from said imaging system; and
introducing a fluorescent component (70) and a quenching component (72) to the deformable particle, such that the quenching component (70) is separated from the fluorescent component (72) at a distance based on a geometry of the deformable particle, wherein the quenching component (72) is adapted to absorb a portion of electromagnetic radiation emitted from the fluorescent component (70) in response to excitation by an optical imaging system.

## Patentansprüche

1. Kontrastmittel (18) für ein bildgebendes System (10), umfassend:
ein verformbares Teilchen mit einer Geometrie, die in Reaktion auf eine Emission des bildgebenden Systems variiert, wobei das verformbare Teilchen umfasst:
eine Hülle (66) mit einer darin befindlichen Substanz (68);
eine fluoreszierende Komponente (70), die angepasst ist, um elektromagnetische Strahlung (28) abzugeben;
und
eine Quenchkomponente (72), die basierend auf der Geometrie von der fluoreszierenden Komponente (70) beabstandet ist, wobei die Quenchkomponente (72) angepasst ist, um einen Teil der elektromagnetischen Strahlung (28) von der fluoreszierenden Komponente (70) zu absorbieren.

2. Kontrastmittel (18) nach Anspruch 1, wobei die Hülle (66) eine Vielzahl an fluoreszierenden Komponenten (70) und Quenchkomponenten (72) umfasst.

3. Kontrastmittel (18) nach Anspruch 1, wobei die in der Hülle befindliche Substanz (68) die fluoreszierende Komponente (70) und die Quenchkomponente (72) umfasst.

4. Kontrastmittel (18) nach Anspruch 1, wobei die Hülle (66) die fluoreszierende Komponente (70) oder die Quenchkomponente (72) und die darin befindliche Substanz (68) die entsprechend andere Komponente umfasst.

5. Bildgebendes Verfahren mit kombinierten Modalitäten (10), umfassend:
ein erstes bildgebendes Gerät einer ersten Modalität (12); und
ein zweites bildgebendes Gerät einer zweiten Modalität (14), die sich von der ersten Modalität (12) unterscheidet, wobei das erste und das zweite bildgebende Gerät angepasst sind, um mit einem Kontrastmittel (18) zu interagieren, das angepasst ist, um von einem Patienten (16) aufgenommen zu werden, wobei das Kontrastmittel (18) umfasst:
eine Hülle (66) mit einer darin befindlichen Substanz (68);
ein verformbares Teilchen mit einer Geometrie, die in Reaktion auf eine Emission des ersten bildgebenden Systems variiert, wobei das verformbare Teilchen umfasst:
eine fluoreszierende Komponente (70), die angepasst ist, um elektromagnetische Strahlung (28) abzugeben, die von dem zweiten bildgebenden System erfasst werden kann; und
eine Quenchkomponente (72), die basierend auf der Geometrie von der fluoreszierenden Komponente (70) beabstandet ist, wobei die Quenchkomponente (72) angepasst ist, um einen Teil der elektromagnetischen Strahlung (28) zu absorbieren.

6. Bildgebendes Verfahren mit kombinierten Modalitäten (10) nach Anspruch 5, wobei das erste bildgebende Gerät ein Ultraschallbildgebungsgerät (30) umfasst.

7. Bildgebendes Verfahren mit kombinierten Modalitäten (10) nach Anspruch 5, wobei das zweite bildgebende Gerät ein optisches bildgebendes Gerät (44) umfasst.

8. Verwendung des Kontrastmittels (18) nach Anspruch 1 in dem bildgebenden Verfahren mit kombinierten Modalitäten nach Anspruch 6, wobei die Verwendung umfasst:
Gewähren von ausreichend Zeit, damit das Kontrastmittel (18) durch einen Patienten (16) an einen interessierenden Bereich (22) in dem Patienten (16) fließen kann;
Abgeben elektromagnetischer Strahlung (28) von der fluoreszierenden Komponente (70) in Reaktion auf die Emissionen eines auf elektromagnetischer Strahlung basierenden bildgebenden Gerätes;
Erhöhen der Geometrie des verformbaren Teilchens in Reaktion auf eine Druckwelle (24) durch ein Ultraschallbildgebungsgerät (30); und
bei der Quenchkomponente (72) nachlassendes Absorbieren eines Teils der elektromagnetischen Strahlung (28), die in Reaktion auf das Erhöhen der Geometrie des verformbaren Teilchens von der fluoreszierenden Komponente (70) abgegeben wird.

9. Verfahren zur Herstellung eines Kontrastmittels (18) für ein bildgebendes System, wobei das Verfahren umfasst:
Bilden einer Hülle (66) um einen temporären Kern;
Stabilisieren der Hülle (66);
Entfernen des temporären Kerns, um ein verformbares Teilchen eines Kontrastmittels (18) zu bilden, wobei das verformbare Teilchen eine Geometrie aufweist, die in Reaktion auf eine Emission des bildgebenden Systems variiert; und
Einführen einer fluoreszierenden Komponente (70) und einer Quenchkomponente (72) in das verformbare Teilchen, so dass die Quenchkomponente (70) basierend auf der Geometrie des verformbaren Teilchens von der fluoreszierenden Komponente (70) beabstandet ist, wobei die Quenchkomponente (72) angepasst ist, um einen Teil der von der fluoreszierenden Komponente (70) abgegebenen elektromagnetischen Strahlung in Reaktion auf die Anregung durch ein optisches bildgebendes System zu absorbieren.

## Revendications

1. Agent de contraste (18) pour un dispositif d'imagerie (10), comportant :
une particule pouvant se déformer, présentant une géométrie qui varie en réponse à une émission à partir du dispositif d'imagerie, la particule pouvant se déformer comprenant :
une enveloppe (66) comportant une substance interne (68) ;
un composant fluorescent (70) adapté de manière à émettre un rayonnement électromagnétique (28) ; et
un composant d'extinction (72) séparé du composant fluorescent (70) à une distance en fonction de la géométrie, dans lequel le composant d'extinction (72) est adapté de manière à absorber une partie du rayonnement électromagnétique (28) issu du composant fluorescent (70).

2. Agent de contraste (18) selon la revendication 1, dans lequel l'enveloppe (66) comprend une multitude du composant fluorescent (70) et du composant d'extinction (72).

3. Agent de contraste (18) selon la revendication 1, dans lequel la substance interne (68) comprend le composant fluorescent (70) et le composant d'extinction (72).

4. Agent de contraste (18) selon la revendication 1, dans lequel l'enveloppe (66) comprend l'un du composant fluorescent (70) ou du composant d'extinction (72) et la substance interne (68) comprend le composant fluorescent (70) ou le composant d'extinction (72) restant.

5. Dispositif d'imagerie à modalité mixte (10), comprenant :
un premier dispositif d'imagerie d'une première modalité (12) ; et
un second dispositif d'imagerie d'une seconde modalité (14) différente de la première modalité (12), dans lequel les premier et second dispositifs d'imagerie sont tous les deux adaptés pour interagir avec un agent de contraste (18) adapté de manière à être reçu par un sujet (16), l'agent de contraste (18) comportant :
une enveloppe (66) présentant une substance interne (68);
une particule pouvant se déformer présentant une géométrie qui varie en réponse à une émission à partir du premier dispositif d'imagerie, la particule pouvant se déformer comportant :
un composant fluorescent (70) adapté de manière à émettre un rayonnement électromagnétique (28) détectable par le second dispositif d'imagerie ; et
un composant d'extinction (72) séparé du composant fluorescent (70) à une distance fonction de la géométrie, dans lequel le composant d'extinction (72) est adapté de manière à absorber une partie du rayonnement électromagnétique (28).

6. Dispositif d'imagerie à modalité mixte (10) selon la revendication 5, dans lequel le premier dispositif d'imagerie comprend un dispositif d'échographie (30).

7. Dispositif d'imagerie à modalité mixte (10) selon la revendication 5, dans lequel le deuxième dispositif d'imagerie comprend un dispositif d'imagerie optique (44).

8. Utilisation de l'agent de contraste (18) selon la revendication 1, dans le dispositif d'imagerie à modalité mixte selon la revendication 6, ladite utilisation comprenant :
l'attente d'une durée suffisante afin d'assurer l'écoulement dudit agent de contraste (18) à travers un sujet (16) jusqu'à une région d'intérêt (22) sur ledit sujet (16) ;
l'émission d'un rayonnement électromagnétique (28) à partir du composant fluorescent (70) en réponse à des émissions à partir d'un dispositif d'imagerie basé sur un rayonnement électromagnétique ;
l'augmentation de la géométrie de la particule pouvant se déformer en réponse à une onde de pression (24) par un dispositif d'échographie (30) ; et
l'absorption de manière décroissante, avec le composant d'extinction (72), d'une partie du rayonnement électromagnétique (28) émis par le composant fluorescent (70) en réponse à l'augmentation de la géométrie de la particule pouvant se déformer.

9. Procédé de fabrication d'un agent de contraste (18) pour un dispositif d'imagerie, le procédé comprenant :
la construction d'une enveloppe (66) autour d'un noyau provisoire;
la stabilisation de l'enveloppe (66) ;
l'élimination du noyau provisoire afin de former une particule pouvant se déformer d'un agent de contraste (18) ladite particule pouvant se déformer présentant une géométrie qui varie en réponse à une émission à partir dudit dispositif d'imagerie ; et
l'introduction d'un composant fluorescent (70) et d'un composant d'extinction (72) dans la particule pouvant se déformer, de telle sorte que le composant d'extinction (70) ou (72) soit séparé du composant fluorescent (70) ou (72) d'une distance basée sur une géométrie de la particule pouvant se déformer, dans lequel le composant d'extinction (72) est adapté de manière à absorber une partie du rayonnement électromagnétique émis à partir du composant fluorescent (70) en réponse à une excitation par un dispositif d'imagerie optique.
